Europäisches Patentamt

European Patent Office

Office européen des brevets

(19)

(11) Veröffentlichungsnummer: **0 008 720**
**B1**

(12)  **EUROPÄISCHE PATENTSCHRIFT**

(45) Veröffentlichungstag der Patentschrift: **23.06.82**

(51) Int. Cl.³: **G 01 N 33/54, C 12 N 5/00**

(21) Anmeldenummer: **79102976.2**

(22) Anmeldetag: **16.08.79**

(54) Diagnostisches Mittel und Verfahren zum Nachweis von antimitochondrialen oder antinukleären Antikörpern.

(30) Priorität: **19.08.78 DE 2836362**

(43) Veröffentlichungstag der Anmeldung:
**19.03.80 Patentblatt 80/6**

(45) Bekanntmachung des Hinweises auf die Patenterteilung:
**23.06.82 Patentblatt 82/25**

(84) Benannte Vertragsstaaten:
**AT BE CH DE FR GB IT LU NL SE**

(56) Entgegenhaltungen:
**DE - A - 2 447 700**
**DE - A - 2 608 747**
**US - A - 4 017 361**

**DEUTSCHE MEDIZINISCHE WOCHENSCHRIFT, Band 98, Nr. 24, 1973 Stuttgart J. SENNEKAMP et al. "Simultaner Kern- und Mitochondrien-Antikörpernachweis in der Immunfluoreszenz" Seiten 1223 bis 1224**

(73) Patentinhaber: **BEHRINGWERKE AKTIENGESELLSCHAFT**
**Postfach 1140**
**D-3550 Marburg/Lahn (DE)**

(72) Erfinder: **Ax, Wolfgang, Prof. Dr.**
**Am Kornacker 76**
**D-3550 Marburg/Lahn (DE)**
Erfinder: **Bauer, Hartwig Wilhelm, Dr.**
**Pfingstrosenstrasse 89**
**D-8000 München 70 (DE)**
Erfinder: **Sedlacek, Hans-Harald, Dr.**
**Sonnenhang 3**
**D-3550 Marburg/Lahn (DE)**

(74) Vertreter: **Meyer-Dulheuer, Karl-Hermann, Dr. et al,**
**HOECHST Aktiengesellschaft Zentrale Patentabteilung Postfach 80 03 20**
**D-6230 Frankfurt/Main 80 (DE)**

Diagnostisches Mittel und Verfahren zum Nachweis von antimitochondrialen oder antinukleären Antikörpern

Die Erfindung betrifft ein Diagnostikum zum Nachweis antimitochondrialer und antinukleärer Antikörper.

Autoimmune Erkrankungen sind gekennzeichnet durch das Auftreten von Antikörpern gegen körpereigenen Zellbestandteile wie z.B. Kernproteine, DNS, Mitochondrien.

Die Bedeutung der antinukleären und antimitochondrialen Faktoren liegt in der Diagnostik und Verlaufskontrolle von Autoimmunphänomenen wie chronischen Lebererkrankungen, Malignomen, Arzneimittelintoxikationen und Kollagenosen.

Diagnostika zum Nachweis antinukleärer Antikörper sind bereits bekannt. Ebenso ist bekannt ein Diagnostikum zum Nachweis antimitochondrialer Antikörper. Ihr Prinzip ist die indirekte oder Doppelantikörpermethode mit Gefrierschnitten von Leber oder Niere oder Zellen von Blut oder Organen unterschiedlicher Spezies als Substrat und mit markierten Antikörpern, gerichtet gegen Antigene Determinanten der gesuchten Antikörper als diagnostische Antikörper (Nakumura, R. M., Chisari, F. V.; Edgingbon, T. S. (1975) Laboratory tests for diagnosis of autoimmune disease. In: M. Stefanini: Progress in Clinical Pathology, pp 177—203, Grune and Stratton, New York). Ihr Nachteil is ihre geringe Haltbarkeit. Man ist also gezwungen, fortlaufend neue Präparate aus frisch gewonnenen Organen bzw. Zellen herzustellen. Ein weiterer Nachteil ist die geringe Antigenmenge in den verschiedenen Präparaten, welche eine exakte Diagnose erschwert.

Es besteht demnach ein Bedarf an einem Diagnostikum, welches die vorgenannten Nachteile nicht besitzt und die Bestimmung beider Arten von Antikörpern gestattet.

Überraschenderweise wurde nun gefunden, daß sich hierfür zellen des Hypernephroms besonders eignen.

Gegenstand der Erfindung ist demnach ein Diagnostikum zum Nachweis sowohl antimitochondrialer als auch antinukleärer Antikörper, dadurch gekennzeichnet, daß es im wesentlichen aus in Zellkultur gezüchteten Hypernephromzellen besteht, die sich fixiert oder getrocknet auf einem Objektträger befinden sowie die betreffenden diagnostischen Verfahren, die als Substratzellen Hypernephromzellen verwenden.

Derartige Zellen können in Zellkultur vermehrt werden, beispielsweise nach den ursprünglich von R. Dulbecco und M. Vogt (J. Exp. Med. 99: 167, 1954), C. Rappaport (Bull. World Health Organ. 14: 147, 1956) und E. Y. Lasfargue (Exper. Coll. Res. 13: 553, 1957) beschriebenen Methoden.

Die Zellen wachsen epithelartig, breiten sich dabei flach aus und bilden einen an der Oberfläche von Kulturgefäßen haftenden einschichtigen Zellrasen. Die Zellen können auch auf Objektträgern direkt vermehrt werden, auf denen sich gleichfalls ein Zellrasen ausbildet.

Die Kerne der Zellen sind im Vergleich zu anderen bekannten Gewebekulturzellen deutlich sichtbar, einschließlich der Nukleoli. Diese Eigenschaft macht sie besonders zum Nachweis antinukleärer Antikörper geeignet.

Die Zellen zeichnen sich weiter dadurch aus, daß sie sehr große, langgestreckte Mitochondrien besitzen, welche sie für den Nachweis antimitochondrialer Antikörper geeignet macht.

Erfindungsgemäß geeignete Zellen können aus einem Nierentumor beispielsweise wie folgt gewonnen werden:

Möglichst frisches Operationsmaterial eines Nierentumors, welches unter sterilen Bedingungen gewonnen und in physiologischem Milieu transportiert wurde, wird mechanisch zerkleinert, enzymatisch aufgeschlossen und die Hypernephromzellen in Gewebekultur vermehrt. Dazu werden vorteilhaft die durch Operation gewonnenen Gewebestückchen (ca 1 mm$^3$) in Calcium- und Magnesium-freier Salzlösung aufgenommen und durch Rühren mit einem Magnetrührer gewaschen. Die Waschflüssigkeit wird verworfen. Eine Lösung von Kollagenase in physiologischer Salzlösung ohne Calcium und Magnesium wird den Stückchen anschließend zugesetzt und ca. 15 Min. gerührt. Der Überstand wird gewonnen und eine 0,25% Trypsinlösung in Calcium- und Magnesiumfreiem Milieu, temperiert auf 37°C, den Stückchen zugesetzt und ca. 15 Min gerührt. Der Überstand wird gewonnen und das Sediment erneut 15 Min. mit der Trypsinlösung gerührt und ebenfalls der Überstand gewonnen. Dieser Prozeß kann mehrfach wiederholt werden. Die Überstände werden jeweils bei 200×g zentrifugiert und in Gewebekulturmedium mit Zusatz von 20% foetalem Kalberserum aufgenommen.

Je 10 ml dieser so gewonnenen Zellsuspension werden als Primärkultur in Petrischalen oder Schraubdeckelflaschen, insbesondere solcher aus Kunststoff, ausgesät. Die Zellkonzentration soll $5 \times 10^5$ Zellen pro Milliliter betragen. Das Anzüchten in Petrischalen erfolgt in feuchter Atmosphäre bei 37° und pH-Regulierung durch $CO_2$-Zufuhr.

Die Zellen werden wie folgt vermehrt:

Nachdem sich ein geschlossener oder nahezu geschlossener Zellrasen in den Primärkulturen gebildet hat, werden die Zellen aus dem Zellrasen der Primärkultur durch eine Enzymbehandlung mit Trypsinlösung herausgelöst, in frischem Gewebekulturmedium suspendiert und wieder in neue Kulturgefäße ausgesät. Durch entsprechende Verdünnung der zu propagierenden Zellsuspension entstehen aus einer Petrischale mit geschlossenem Zellrasen (Monolayer) mindestens 3 Tochterkulturen. Diese 3 Tochterkulturen der ersten Passage sind, zweckmäßig in Petrischalen, nach 2—3 Tagen wiederum so dicht bewachsen, daß sie umgesetzt werden können.

Während Primärkulturen in Petrischalen aus Kunststoff (10 cm Durchmesser) oder Schraubverschlußflaschen aus Kunststoff (10 ml nutzbarer Inhalt) angezüchtet werden können, lassen sich größere Zellmengen in geeigneten größeren Flaschen aus Glas oder Kunststoff und anderen Vorrichtungen zur Massenkultur herstellen.

In den für diagnostische Zwecke geeigneten Mengen werden die Zellen auf Glas- oder Kunststoffflächen wie Deckgläser, Objektträger oder am Boden der Näpfchen einer Mikrokulturschale etabliert.

Die Hypernephromzellen können mühelos bis in die 50. Passage und darüber hinaus geführt werden. Sie lassen sich nach bekannten Verfahren in flüssigem Stickstoff bei —196°C lagern und vital erhalten.

Für den Nachweis antinukleärer Antikörper empfiehlt sich eine Fixierung der Zellen. Sie wird mit bekannten Fixationsmitteln, z.B. Formalin, Aceton, Methanol, Aethanol bzw. deren Gemischen, vorzugsweise mit Aceton in literaturbekannter Weise durchgeführt, J. Lenng Tack et al., Arch. Derm. Forsch. 247, 161—170, (1973).

Für den Nachweis von antimitochondrialen Antikörpern hat eine Fixierung zu unterbleiben, da sie die korrespondierenden antigenen Determinanten an den Mitochondrien zerstört.

Der Nachweis der antimitochondrialen bzw. der antinukleären Antikörper wird wie folgt durchgeführt.

## 1. Nachweis antimitochondrialer Antikörper

Die mit luftgetrockneten, nicht fixierten Hypernephromzellen beschichteten Objektträger werden mit dem zu prüfenden Antikörper, gelöst in einer beliebigen isotonischen Salzlösung, z.B. 0,9% iger, phosphatgepufferter NaCl-Lösung, überschichtet und für 10 Min bis 2 Stunden, vorzugsweise 30 Minuten, in einer feuchten Kammer inkubiert. Nachfolgend wird ausgiebig möglichst 1 bis 6 mal, vorzugsweise 3 mal in einer isotonischen Salzlösung gewaschen, sodann überschichtet man die Objektträger mit einem fluoreszenzmarkierten Antikörper, gerichtet gegen Antigene-Determinanten des gesuchten Antikörpers in einer Verdünnung, durch die unspezifische Reaktionen mit den Hypernephromzellen ausgeschlossen sind. Es wird, in einer feuchten Kammer 10 Min. bis 2 Stunden, vorzugsweise 30 Min. inkubiert, nachfolgend wiederum 1 bis 6 mal, vorzugsweise 3 mal gewaschen und letztlich luftgetrocknet. Die letzte Waschung sollte mit demineralisiertem Wasser durchgeführt werden.

Die mikroskopische Auswertung des derartig behandelten Präparates erfolgt mit dem Fachmann bekannten Methoden der Immunfluoreszenzmikroskopie, z.B. nach Wick, Baudner, Herzog, Immunfluoreszenz, Beiträge zur Theorie und Praxis, Medizinische Verlagsgesellschaft mbH, Marburg, 1976.

## 2. Nachweis antinukleärer Antikörper

Der Nachweis von antinukleären Antikörpern erfolgt entsprechend wie der Nachweis von antimitochondrialen Antikörpern. Jedoch sind hier vorzugsweise fixierte anstelle von luftgetrockneten nichtfixierten Hypernephromzellen zu verwenden.

Eine geeignete Methode stellt die Fixation mit Aceton dar (Gell, Coombs, Jachmann, Clinical aspects of immunology, Blackwell 1975 p 1122).

Die Erfindung wird am nachstehenden Beispiel näher erläutert:

Beispiel

Tumorgewebe eines Hypernephroms (hypernephroides Karzinom) wird unter sterilen Bedingungen während der Operation gewonnen und in Gewebekulturmedium aufgefangen. Letzteres ist ein sogenanntes Eagle's Medium in der Dulbecco-Modifikation mit der gebräuchlichen Zusammensetzung:

| Aminosäuren | mg/l |
|---|---|
| L-Arginin HCl | 84,0 |
| Glycin | 30.0 |
| L-Cystin | 48.0 |
| L-Histidin HCl . $H_2O$ | 42.0 |
| L-Isoleucin | 105.0 |
| L-Leucin | 105.0 |
| L-Lysin HCl | 146.0 |
| L-Methionin | 30.0 |
| L-Phenylalanin | 66.0 |
| L-Serin | 42.0 |
| L-Threonin | 95.0 |
| L-Tryptophan | 16.0 |
| L-Tyrosin | 72.0 |
| L-Valin | 94.0 |

| Vitamine | mg/l |
|---|---|
| D-Ca-Pantothenat | 4.00 |
| Cholin-Chlorid | 4.00 |
| Folsäure | 4.00 |
| i-Inositol | 7.20 |
| Nicotinamid | 4.00 |
| Pyridoxal-HCl | 4.00 |
| Riboflavin | 0.40 |

| Anorganische Salze und sonstige Zusätze | |
|---|---|
| $CaCl_2$ | 200.00 |
| KCl | 400.00 |
| $MgSO_4 . 7H_2O$ | 200.00 |
| NaCl | 6400.00 |
| $NaHCO_3$ | 3700.00 |
| $NaH_2PO_4 . 2H_2O$ | 124.00 |
| $Fe(NO_3)_3 . 9H_2O$ | 0.10 |
| Glucose | 4500.00 |
| Phenolrot | 15.00 |
| Natriumbicarbonat | 3.700 |
| Penicillin | 10.000 IE |
| Streptomycin | 10.000 $\mu g$ |

Das Tumorgewebe wird innerhalb weniger Stunden weiterverarbeitet. Dabei wird zunächst das nicht zu dem Hypernephromgewebe gehörende Gewebe weitgehend entfernt und nur möglichst reines, nicht zerstörtes Tumorgewebe präpariert, dies wiederum in Gewebekulturmedium unter sterilen Bedingungen, was auch für alle folgenden Schritte gilt. Etwa 1 cm³ des Tumorgewebes wird anschließend mechanisch weiter zerkleinert zu Stückchen von etwa 1 mm Kantenlänge und letztere in 30 ml Puck's Salzlösung (Puck's Saline A) aufgenommen.

Puck's Salzlösung hat die Zusammensetzung:

| | |
|---|---|
| NaCl | 8,00 g |
| KCl | 0,40 g |
| NaHCO₃ | 0,35 g |
| Glucose | 1,00 g |

auf 1 Liter destilliertes Wasser.

Die ab hier verwendeten Lösungen sind auf 37°C temperiert.

Die Suspension wird in einer Trypsinierflasche nach Rappaport (Bull. World Health Organ. 14: 147, 1956; Hersteller: Bellco Glass Inc., Vineland N.J., USA, Kat. Nr. 1996) mit einem Magnetrührer für 5 Min gerührt. Der Überstand wird verworfen und 30 ml Kollagenase-Lösung (10 mg Kollagenase, Serva Heidelberg, auf 20 ml PBS ohne Ca und Mg, Zusammensetzung: NaCl 8,0 g, KCl 0,2 g Na₂HPO₄ . 12H₂O 2,9 g, KH₂PO₄ 0,2 g auf 1 Liter destilliertes Wasser) zugesetzt. Es wird für 15 Min. gerührt. Der Überstand wird gewonnen, indem er in ein Zentrifugenglas dekantiert wird. 30 ml einer 0,25% Trypsinlösung werden dem Sediment zugegeben und für 15 Min gerührt. Zusammensetzung der Trypsinlösung: 5 ml Trypsin (Difco 1:250) 5% ig auf 100 ml Puck's Saline A, pH 8—8,5. Es wird 15 Min. gerührt und der Überstand gewonnen und nochmals mit Trypsinlösung gerührt. Die Überstände werden jeweils bei 200×g zentrifugiert und das so gewonnene Zellsediment in 20 ml Dulbecco's Medium (s.o.) mit einem Zusatz von 20% fetalem Kälberserum suspendiert. Die Zellkonzentration wird auf 5×10⁵ Zellen im Milliliter eingestellt und anschließend je 10 ml der Suspension in geeignete Petrischalen aus Polystyrol-Kunststoff (Durchmesser 10 cm, Fa. Greiner, Nürtingen) eingesät. Die Schalen werden bei 37°C in feuchter Atmosphäre in einem CO₂-begasten Brutschrank inkubiert.

Nach Anwachsen der Primärkultur mit der Ausbildung eines geschlossenen einschichtigen Zellrasen (Monolayer) werden die Zellen der Primärkultur mit Hilfe der obengenannten Trypsinlösung von dem Boden der Petrischale und aus ihrem Verband gelöst, in Gewebekulturmedium aufgenommen, auf die gewünschte Zellzahl verdünnt und auf neue Petrischalen verteilt. Aus einer bewachsenen Petrischale gehen 3—4 neue Schalen der nächsten Passage hervor. Die Verdoppelungszeit der Zellen liegt bei 18 Stunden. Das Medium in den Kulturschalen wird alle 2—3 Tage erneuert.

Nach erfolgreicher Propagierung über 10 Passagen können Objektträgerkulturen angelegt werden. Dazu werden Zellen durch Trypsinieren aus Kulturen gewonnen, in Gewebekulturmedium auf eine Konzentration von 1—2×10⁶ Zellen pro ml eingestellt und tropfenweise (1 Tropfen etwa 20 µl) auf vorbereitete Objektträger so verteilt, daß jeweils 1 Tropfen in genügendem Abstand vom nächsten sich befindet. Beispielsweise dienen dazu Objektträger mit einem lichtundurchlässigen schwarzen Farbüberzug, welcher kreisförmige Aussparungen enthält, in deren Bereich die Zellen zu etablieren sind.

Die Zellen aus einem Tropfen siedeln sich also im Bereich dieses Tropfens an, indem sie in diesem absinken und auf dem Glas des Objektträgers sich ausbreiten und festwachsen. Dabei muß der Objektträger in einer feuchtegesättigten, CO₂-begasten Atmosphäre bei 37°C für 4—6 Stunden ohne Bewegung gehalten werden.

Danach wird der Objektträger für weitere 12—18 Stunden in Gewebekulturmedium untergetaucht weiterkultiviert. Es bildet sich in der Regel ein kreisförmiger Rasen von Hypernephromzellen mit Durchmesser von 4—5 mm aus.

Nachfolgend werden die Objektträger dem Gewebekulturmedium entnommen und bei Zimmertemperatur luftgetrocknet.

A. Nachweis von antimitochondrialen Antikörpern

Das zu prüfende, möglicherweise antimitochondriale Antikörper enthaltende Serum wird mit physiologischer Kochsalzlösung, enthaltend 0,1% Albumin, in einer geometrisch abgestuften Reihe verdünnt (z.B. 1:1, 1:2, 1:4, 1:8, 1:16, 1:32, 1:64, 1:128). Ein Tropfen der jeweiligen Verdünnung wird auf jeweils einen, an dem Objektträger haftenden kreisförmigen Zellrasen geschichtet. Der mit unterschiedlichen Verdünnungen derart beschichtete Objektträger wird in einer feuchten Kammer (dampfundurchlässiger Kasten, dessen Boden mit Flüssigkeit bedeckt ist, über deren Oberfläche sich ein Gitter zur horizontalen Lagerung der Objektträger befindet) für 30 Minuten bei Zimmertemperatur (20°C) gelagert, nachfolgend durch Eintauchen in ein Gefäß, enthaltend physiologische Kochsalzlösung mit 0,1% Albumin, Herausnahme nach 5 Minuten und entsprechendes Eintauchen in ein zweites und drittes Gefäß dreimal gewaschen. Nach Ablaufen der anhaftenden Flüssigkeit durch kurzzeitiges (ca. 5 Minuten) Lagern in Schrägstellung werden die Zellrasen des Objektträgers mit einem Tropfen eines mit Fluorescein-Isothiocyanat (FITC) markierten Antikörpers vom Kaninchen, gerichtet gegen menschliche Immunglobuline, überschichtet. Anschließend werden die Objektträger erneut bei 20°C in der feuchten Kammer gelagert und nachfolgend wie oben beschrieben dreimal gewaschen. Als Arbeitsverdünnung des FITC-markierten Antikörpers wird eine Verdünnung gewählt, welche allein auf Zellen aufgelagert diese nicht unspezifisch färbt. Nach Lufttrocknung werden die Zellrasen mit Glycerin (frei von Eigenfluoreszenz) und mit einem Deckglas abgedeckt und mit einem Fluoreszenzmikroskop, Fa. Zeiss, in Auflicht bei Wahl eines 25-fach, 40-fach und 100-fach vergrößernden Objektives mikroskopisch beurteilt.

Als positiv gelten solche Prüfseren, welche in dem vorbeschriebenen Versuchsansatz zur Fluoreszenz der Mitochondrien der Hypernephromzellen führen.

B. Nachweis von antinukleären Antikörpern

Die mit kreisförmigen Rasen beschichteten Objektträger werden für 10 Minuten in Aceton eingetaucht, nachfolgend dreimal wie unter A. beschrieben mit physiologischer Kochsalzlösung, enthaltend 0,1% Albumin, gewaschen und luftgetrocknet. Die Verdünnung des antinukleäre Antikörper enthaltenden Serums, Aufschichtung auf den Objektträger, Inkubation in der feuchten Kammer, Waschung, Aufschichtung mit dem FITC-markierten Antikörper, Inkubation, Waschung, Lufttrocknung, Eindeckelung und mikroskopische Untersuchung geschieht wie unter A. beschrieben.

Als positiv gelten solche Prüfseren, in welchen die Zellkerne entweder homogen oder unterschiedlich gefleckt oder die Kernmembran oder das Kernkörperchen fluoreszieren.

**Patentansprüche**

1. Diagnostisches Mittel zum Nachweis von antimitochondrialen oder antinukleären Antikörper, dadurch gekennzeichnet, daß es im wesentlichen aus in Zellkultur gezüchteten Hypernephromzellen besteht, die sich fixiert oder getrocknet auf einem Objektträger befinden.

2. Verfahren zum Nachweis von antimitochondrialen oder antinukleären Antikörpern in einer Immunfluoreszenzmethode mit indirekter Markierung mittels Zellen als Substrat, dadurch gekennzeichnet, daß man als Zellen Hypernephromzellen verwendet.

**Claims**

1. Diagnostic agent for testing for the presence of anti-mitochondrial or anti-nuclear antibodies essentially comprising in cell culture cultivated hypernephroma cells which are fixed or dried on a microscope slide.

2. Process for testing for the presence of anti-mitochondrial or anti-nuclear antibodies in an immunofluorogenic method with indirect labelling using cells as a substrate which comprises using hypernephorma cells as a substrate.

**Revendications**

1. Agent de diagnostic pour la détection d'anticorps antimitochondries ou antinucléaires, caractérisé en ce qu'il consiste essentiellement en des cellules d'hypernéphrome cultivées dans une culture cellulaire qui sont fixées ou séchées sur un porte-objets.

2. Procédé de détection d'anticorps antimitochondries ou antinucléaires par une méthode d'immunofluorescence par marquage indirect au moyen de cellules utilisées comme substrat, caractérisé en ce qu'on utilise comme cellules des cellules d'hypernéphrome.